# EUROPÄISCHE PATENTSCHRIFT

(11) **EP 2 229 872 B1**
(45) Veröffentlichungstag und Bekanntmachung des Hinweises auf die Patenterteilung: **10.08.2011**
(21) Anmeldenummer: 10002513.9
(22) Anmeldetag: 10.03.2010
(51) Int. Cl.: A61B 1/307

(54) **Uretero-Renoskop**
Ureterorenoscope
Urétéro-rénoscope

(30) Priorität: 18.03.2009 DE 102009013312
(43) Veröffentlichungstag der Anmeldung: 22.09.2010
(73) Patentinhaber: Richard Wolf GmbH, 75438 Knittlingen (DE)
(72) Erfinder: Heimberger, Rudolf, 75038 Oberderdingen (DE)
(74) Vertreter: Vollmann, Heiko

(56) Entgegenhaltungen:
- EP-A1- 1 629 764
- EP-A1- 1 743 567
- WO-A2-2008/106679
- DE-A1- 19 826 311
- JP-A- 10 309 259

## Beschreibung

Die Erfindung betrifft ein Uretero-Renoskop mit den im Oberbegriff des Anspruchs 1 angegebenen Merkmalen.

Uretero-Renoskope dienen zur transurethralen Diagnostik und Therapie im Harnleiter- und Nierenbeckenbereich. Häufig werden diese Instrumente zur Entfernung von Harnsteinen aus dem Harnleiter oder den Nierenkelchgruppen eingesetzt. Hierzu wird der meist flexibel ausgebildete Hohlschaft des Uretero-Renoskops ausgehend von der Harnleitermündung bis zu dem Harnstein in den Harnleiter eingeführt, wo der Harnstein entweder direkt mit einem durch den Hohlschaft geführten Steinfasskörbchen entnommen werden kann oder im Falle größerer Steine zuvor beispielsweise mittels einer Lasersonde zerkleinert wird. Insbesondere bei sehr englumigen Harnleitern oder bei Harnleiterverengungen, wie z. B. Harnleiterstrikturen, besteht die Gefahr, dass die den Harnleiter umgebende Schleimhaut beim Einführen des Uretero-Renoskops traumatisiert wird oder sogar perforiert wird.

Aus EP 1 629 764 A1 ist ein Instrumentenkopf bekannt, bei dem an einer distalen Stirnseite ein Optikkanal und zwei Beleuchtungskanäle münden. An einer abgeschrägten Umfangsseite mündet ein Instrumentenkanal.

Vor diesem Hintergrund liegt der Erfindung die Aufgabe zugrunde, ein Uretero-Renoskop zu schaffen, das in dem Harnleiter gewebeschonender bewegbar ist und gleichzeitig die Arbeit eines das Uretero-Renoskop nutzenden Operateurs im Vergleich zu bekannten Instrumenten dieser Art erleichtert.

Gelöst wird diese Aufgabe durch ein Uretero-Renoskop mit den in Anspruch 1 angegebenen Merkmalen. Vorteilhafte Weiterbildungen dieses Uretero-Renoskops sind in den Unteransprüchen, der nachfolgenden Beschreibung sowie der Zeichnung angegeben.

Das erfindungsgemäße Uretero-Renoskop weist einen Schaft auf. Hierbei handelt es sich um einen Hohlschaft, der sowohl starr als auch flexibel ausgebildet sein kann. Bevorzugt ist allerdings eine flexible Ausgestaltung des Hohlschafts, so dass der Hohlschaft quer zu seiner Längsausdehnung biegbar ist. An dem distalen Ende des Hohlschafts ist ein Instrumentenkopf angeordnet. Dieser Instrumentenkopf kann Teil des Hohlschafts sein oder als ein separates Teil an dem distalen Ende des Hohlschafts festgelegt sein. An dem Instrumentenkopf münden Führungskanäle für eine Beobachtungsoptik und für Beleuchtungsmittel. Darüber hinaus münden an dem Instrumentenkopf auch Instrumentenkanäle für zu Therapiezwecken eingesetzte Instrumente.

In distaler Richtung verjüngt sich der Instrumentenkopf. Dementsprechend weist der Instrumentenkopf an seinem distalen Ende seinen geringsten Querschnitt auf, der sich proximalwärts stufenlos zu einem größten Querschnitt erweitert, wobei dieser größte Querschnitt des Instrumentenkopfs zweckmäßigerweise im Wesentlichen dem Querschnitt des Hohlschafts entspricht. Diese Ausgestaltung des Instrumentenkopfs ist insofern vorteilhaft, als das erfindungsgemäße Uretero-Renoskop im Vergleich zu bislang bekannten Uretero-Renoskopen mit einem sich nicht verjüngenden distalen Ende bei geringerem Widerstand schleimhautschonender in den Harnleiter eindringen und in dem Harnleiter vordringen kann, wobei es den Harnleiter wie ein Dilatator atraumatisch aufweitet. Die Gefahr einer Gewebetraumatisierung bzw. Perforation wird hierdurch erheblich verringert.

Der Instrumentenkopf verjüngt sich an zwei im Wesentlichen diametral voneinander abgewandten Umfangsabschnitten keilförmig. Dementsprechend ist der Instrumentenkopf an zwei direkt voneinander abgewandten Umfangsabschnitten jeweils so abgeschrägt ausgebildet, dass die Mantelfläche in diesen Umfangsabschnitten des Instrumentenkopfes zwei gerade Ebenen bildet, die bezogen auf eine Mittelachse des Instrumentenkopfs so abgeschrägt sind, dass sie in distaler Richtung auf die Mittelachse des Instrumentenkopfs mehr oder weniger spitz zulaufen.

Das erfindungsgemäße Uretero-Renoskop weist zwei Instrumenten kanäle auf, wobei jeweils ein Instrumentenkanal an einem der beiden keilförmig abgeschrägt ausgebildeten Umfangsabschnitte mündet. Diese Ausgestaltung macht das erfindungsgemäße Uretero-Renoskop besonders zur Lithotripsie von Harnsteinen geeignet. Während es bei einem Uretero-Renoskop mit nur einem Instrumentenkanal bei der Zertrümmerung größerer Harnsteine erforderlich ist, eine zum Zertrümmern der Harnsteine verwendete Sonde nach Gebrauch aus dem Instrumentenkanal zu entfernen, um ein Steinfasskörbchen zum Aufnehmen der Harnsteinfragmente zu dem Operationsgebiet führen zu können, gestattet das Vorhandensein von zwei Instrumentenkanälen, die Sonde zum Zertrümmern der Harnsteine in einem der Instrumentenkanäle zu belassen. Demzufolge entfällt ein ansonsten ständig erforderlicher Austausch von Sonde und Steinfasskörbchen in einem Instrumentenkanal, was einen erheblichen Zeitgewinn bei einer Operation zur Harnsteinentfernung bedeutet. Sowohl der Operateur als auch der Patient werden hierdurch wesentlich entlastet.

Zweckmäßigerweise verjüngt sich der Instrumentkopf nicht in einer solchen Weise, dass sein distales Ende einen Scheitelpunkt oder eine Scheitelkante bildet. Stattdessen ist bevorzugt an dem distalen Ende des Instrumentenkopfes eine Stirnfläche ausgebildet, die typischerweise senkrecht zur Mittelachse des Instrumentenkopfes ausgerichtet ist. Das heißt, obwohl sich der Instrumentenkopf distalwärts verjüngt, ist das distale Ende des Instrumentenkopfes aufgrund der Stirnfläche atraumatisch stumpf ausgebildet.

An der das distale Ende des Instrumentenkopfs bildenden Stirnfläche münden bevorzugt der Führungskanal für die Beobachtungsoptik und der Führungskanal für die Beleuchtungsmittel. Dementsprechend sind diese Instrumententeile an einer Stelle angeordnet, an der das Beobachtungsfeld der Beobachtungsoptik und das Beleuchtungsfeld der Beleuchtungsmittel nicht durch andere Bereiche des Instrumentenkopfes eingeschränkt werden kann. Die Führungskanäle für die Beobachtungsoptik und die Beleuchtungsmittel können an der Stirnfläche mittels dort angeordneter lichtdurchlässiger Endfenster und/oder Linsen verschlossen sein. Daneben kann es auch von Vorteil sein, wenn die gesamte Stirnfläche von einer die Optik und die Beleuchtungsmittel überdeckenden Abdeckung gebildet wird, die zumindest in diesen Bereichen lichtdurchlässig ist.

Die beiden Instrumentenkanäle können so angeordnet sein, dass ihr Lichtraumprofil jeweils vollständig im Bereich eines abgeschrägt ausgebildeten Umfangsabschnitts liegt. Um sicherzustellen, dass sich ein durch einen Instrumentenkanal geführtes Instrument im Sicht- und Beleuchtungsfeld der an der distalen Stirnfläche des Instrumentenkopfs angeordneten Optik bzw. Beleuchtungsmittel befindet, sind die Instrumentenkanäle typischerweise möglichst nah zu der Beobachtungsoptik und den Beleuchtungsmitteln anzuordnen. Zu diesem Zweck sind die Instrumentenkanäle bei dem erfindungsgemäßen Uretero-Renoskop vorzugsweise derart angeordnet,

dass sie jeweils die Übergangskante von dem betreffenden abgeschrägten Umfangsabschnitt zu der Stirnfläche des Instrumentenkopfes schneiden.

Um die Dilatationseigenschaften des erfindungsgemäßen Uretero-Renoskops weiter zu verbessern, kann sich der Instrumentenkopf vorteilhafterweise auch an zumindest einem zwischen den keilförmigen Umfangsabschnitten angeordneten Umfangsabschnitt in distaler Richtung verjüngen. Zu diesem Zweck kann dieser Umfangsabschnitt prismatisch gerade abgeschrägt ausgebildet sein oder sich, wie es vorzugsweise vorgesehen ist, konisch verjüngen.

Lasersonden eignen sich besonders gut als Lithotriptor bei der transurethralen Harnsteinentfernung. Auch bei dem erfindungsgemäßen Uretero-Renoskop ist der Einsatz einer durch einen Instrumentenkanal geführten Lasersonde vorgesehen. Vorteilhafterweise ist diese Lasersonde an einem sich proximalseitig an den Schaft des Uretero-Renoskops anschließenden Steuergehäuse festlegbar. Durch diese Maßnahme kann verhindert werden, dass sich die Lasersonde unerwünscht innerhalb des Schafts verschiebt, was bei einem versehentlichen Aktivieren der Lasersonde ggf. zu einer Zerstörung des Schafts führen könnte. Die Fixierbarkeit der Lasersonde an dem Steuerungsgehäuse ist insofern günstig, als das Steuerungsgehäuse per se zur Handhabung des Uretero-Renoskops dient.

Bevorzugt ist die Lasersonde in dem Instrumentenkanal in Längsrichtung des Instrumentenkanals begrenzt verschiebbar. In diesem Zusammenhang kann ein Verschiebeweg der Lasersonde von einer Arbeitsposition außerhalb des Instrumentenkanals in eine Position innerhalb des Instrumentenkanals und umgekehrt vorgesehen sein, wobei die Position innerhalb des Instrumentenkanals vorteilhafter Weise so gewählt ist, dass ein unabsichtliches Aktivieren des Lasers nicht das Instrument zerstört.

Weiter vorteilhaft kann bei dem erfindungsgemäßen Uretero-Renoskop mindestens ein Instrumentenkanal einen Spülkanal bilden. D.h., dieser Instrumentenkanal ist mit einer Spülflüssigkeitsversorgungsleitung verbindbar. Hierzu kann vorzugsweise an dem Steuerungsgehäuse ein entsprechender Anschluss vorgehalten werden. Über diesen Anschluss kann eine Spülflüssigkeit zu dem Operationsgebiet geleitet werden und ggf. von dort wieder zurückgeleitet werden.

Besonders große Spülflüssigkeitsvolumenströme lassen sich dann erzielen, wenn das Uretero-Renoskop zwei Instrumentenkanäle aufweist, die beide einen Spülkanal bilden, wobei die Spülflüssigkeit über beide Instrumentenkanäle zu dem Operationsgebiet geleitet und von dort wieder zurückgeleitet werden kann. Allerdings ist auf diese Weise nur eine intermittierende Spülung möglich. Um eine Dauerspülung schaffen zu können, sieht eine weitere bevorzugte Ausgestaltung des Uretero-Renoskops vor, einen Instrumentenkanal als einen Spülflüssigkeitszulauf und den zweiten Instrumentenkanal als einen Spülflüssigkeitsrücklauf zu verwenden.

Nachfolgend ist die Erfindung anhand eines in der Zeichnung dargestellten Ausführungsbeispiels näher erläutert. In der Zeichnung zeigt:
- Fig. 1: schematisch ein Uretero-Renoskop in einer Seitenansicht mit einem vergrößert dargestellten distalen Schaftende,
- Fig. 2: schematisch einen Instrumentenkopf des Uretero- Renoskops nach Fig. 1 in einer perspektivischen Darstellung,
- Fig. 3: den Instrumentenkopf nach Fig. 2 in einer Frontansicht,
- Fig. 4: den Instrumentenkopf nach Fig. 2 in einer Seitenansicht und
- Fig. 5: den Instrumentenkopf nach Fig. 2 in einer gegenüber Fig. 4 um 90° gedrehten zweiten Seitenansicht.

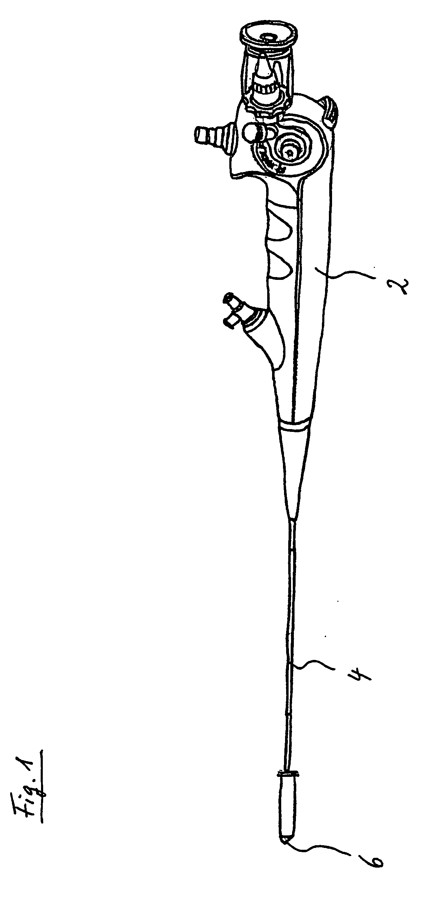

Das in Fig. 1 dargestellte Uretero-Renoskop weist ein zur Handhabung des Instruments dienendes Steuerungsgehäuse 2 auf, an dessen distalen Ende ein Schaft 4 angeordnet ist. Der Schaft 4 ist als ein flexibler Hohlschaft ausgebildet. An dem distalen Ende des Schafts 4 ist ein Instrumentenkopf 6 angeordnet.

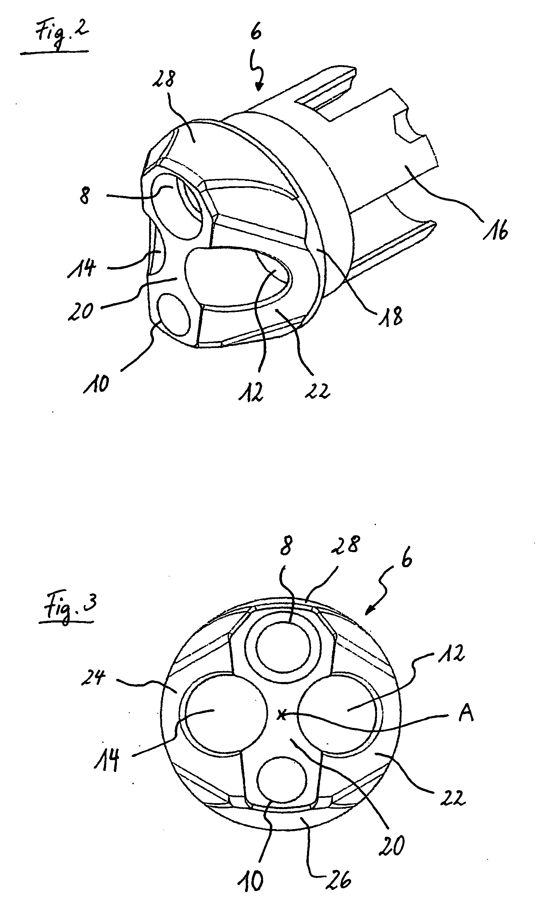

Ausgehend von dem Steuerungsgehäuse 2 erstrecken sich in Fig. 1 nicht erkennbar ein Führungskanal 8 für das Beleuchtungsmittel, ein Führungskanal 10 für eine Beobachtungsoptik sowie zwei Instrumentenkanäle 12 und 14 durch den Schaft 4, wobei die Führungskanäle 8 und 10 sowie die Instrumentenkanäle 12 und 14 alle an dem Instrumentenkopf 6 münden (Figuren 2 und 3). Zur Harnsteinentfernung kann in einem der Instrumentenkanäle 12 und 14 eine nicht dargestellte Lasersonde zum Zertrümmern der Harnsteine geführt sein, die an dem Steuerungsgehäuse 2 festlegbar ist. Der andere Instrumentenkanal 12, 14 kann dann als Zugang für ein ebenfalls nicht dargestelltes Steinfasskörbchen dienen, das durch den Instrumentenkanal 12, 14 zu dem Operationsgebiet geführt werden kann.

Der Instrumentenkopf 6 weist einen Abschnitt 16 auf, der in dem distalen Ende des Schafts 4 unlösbar festgelegt ist. Distalseitig dieses Abschnitts 16 schließt sich unter Ausbildung eines Absatzes 17 ein Abschnitt 18 des Instrumentenkopfs 6 an, der bei festgelegtem Abschnitt 16 in dem Schaft 4 distalseitig außerhalb des Schafts 4 angeordnet. Der Absatz 17 ist so dimensioniert, dass der Instrumentenkopf 6 an dem Absatz 17 den gleichen Außenquerschnitt wie der Schaft 4 aufweist, also dort mit dem Schaft 4 fluchtet.

Im Bereich des Abschnitts 18 verringert sich der Querschnitt des Instrumentenkopfs 6 kontinuierlich in distaler Richtung zu einer Stirnfläche 20, die das distale Ende des Instrumentenkopfes 6 bildet. Insofern bildet der Instrumentenkopf 6 quasi einen Dilatator, mit dem der Harnleiter gewebeschonend aufgeweitet werden kann.

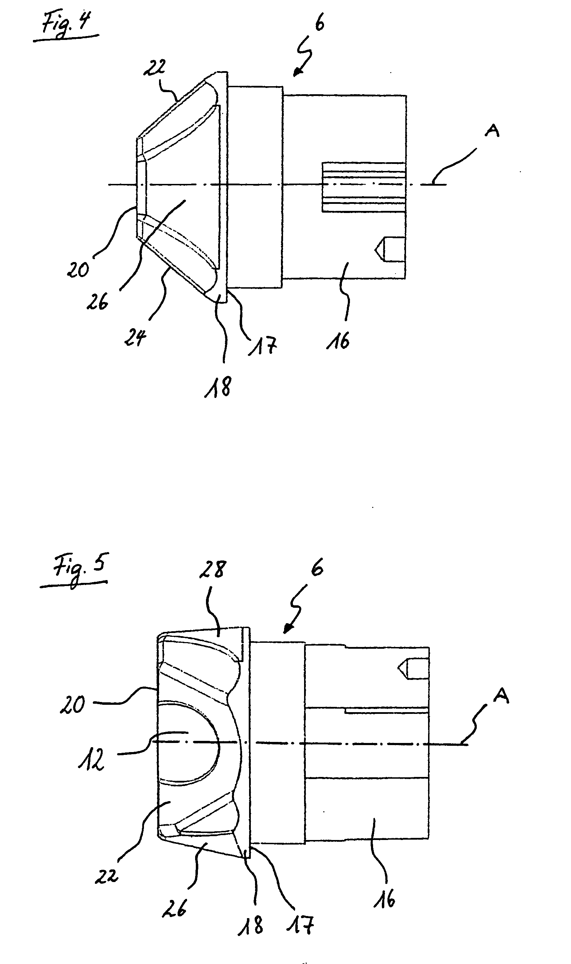

Der Querschnitt der Stirnfläche 20 ist deutlich kleiner als der distale Endquerschnitt des Schafts 4. D.h., der Instrumentenkopf 6 verjüngt sich in Richtung seines distalen Endes bzw. zur Stirnfläche 20 hin. Diese Verjüngung wird im Wesentlichen dadurch hervorgerufen, dass zwei voneinander diametral abgewandte Umfangsabschnitte 22 und 24 des Instrumentenkopfs 6 in Form ebener Flächen in distaler Richtung keilförmig spitz aufeinander zulaufen, wobei sie bezogen auf eine Mittelachse A des Instrumentenkopfs 6 im Wesentlichen den gleichen Neigungswinkel aufweisen (Fig. 4). Darüber hinaus verjüngt sich der Instrumentenkopf 6 auch im Bereich der Umfangsabschnitte 26 und 28, die zwischen den Umfangsabschnitten 22 und 24 liegen. Allerdings erfolgt die Verjüngung des Instrumentenkopfs 6 in den Umfangsabschnitten 26 und 28 konisch bzw. bei gewölbter Mantelfläche dieser Umfangsabschnitte 26 und 28, wobei die Mantelfläche im Bereich des Umfangsabschnitts 26 stärker als im Bereich des Umfangsabschnitts 28 in distaler Richtung auf die Mittelachse A des Instrumentenkopfs 6 zuläuft.

An der Stirnfläche 20 mündet der Führungskanal 8 der Beobachtungsoptik in einem an den Umfangsabschnitt 28 angrenzenden Randbereich, während der Führungskanal 10 für das Beleuchtungsmittel in einem an den Umfangsabschnitt 26 angrenzenden Randbereich der Stirnfläche 20 mündet.

Der Instrumentenkanal 12 ist so angeordnet, dass er hauptsächlich im Bereich des Umfangsabschnitts 22 aus dem Instrumentenkopf 6 austritt, wobei er allerdings die Stirnfläche 20 in einem an dem Umfangsabschnitt 22 angrenzenden Randbereich, der zwischen dem Führungskanal 8 der Beobachtungsoptik und dem Führungskanal 10 der Beobachtungsoptik liegt, schneidet. Korrespondierend hierzu tritt der Instrumentenkanal 14 so aus dem Instrumentenkopf 6 aus, dass seine Öffnung sowohl den Umfangsabschnitt 24 als auch einen daran angrenzenden Randbereich der Stirnfläche 20 zwischen dem Führungskanal 8 der Beobachtungsoptik und dem Führungskanal 10 des Beleuchtungsmittels schneidet. Aufgrund dieser Anordnung der Instrumentenkanäle 12 und 14 in unmittelbarer Nähe des Führungskanals 8 der Beobachtungsoptik und des Führungskanals 10 der Beleuchtungsmittel wird sichergestellt, dass die durch die Instrumentenkanäle 12 und 14 geführten Instrumente in das Beobachtungs- und Beleuchtungsfeld der Beobachtungsoptik und des Beleuchtungsmittels gebracht werden.

### Bezugszeichenliste

- 2: - Steuerungsgehäuse
- 4: - Schaft
- 6: - Instrumentenkopf
- 8: - Führungskanal
- 10: - Führungskanal
- 12: - Instrumentenkanal
- 14: - Instrumentenkanal
- 16: - Abschnitt
- 17: - Absatz
- 18: - Abschnitt
- 20: - Stirnfläche
- 22: - Umfangsabschnitt
- 24: - Umfangsabschnitt
- 26: - Umfangsabschnitt
- 28: - Umfangsabschnitt

- A: Mittelachse

## Patentansprüche

1. Uretero-Renoskop mit einem Schaft (4), an dessen distalen Ende ein Instrumentenkopf (6) angeordnet ist, der sich in distaler Richtung an zwei im Wesentlichen diametral voneinander abgewandten Umfangsabschnitten (22, 24) kontinuierlich keilförmig verjüngt, **dadurch gekennzeichnet, dass** an beiden der keilförmig abgeschrägt ausgebildeten Umfangsflächen (22, 24) je ein Instrumentenkanal (12, 14) mündet.

2. Uretero-Renoskop nach Anspruch 1, **dadurch gekennzeichnet, dass** an dem distalen Ende des Instrumentenkopfes (6) eine Stirnfläche (20) ausgebildet ist.

3. Uretero-Renoskop nach einem der vorangehenden Ansprüche, **dadurch gekennzeichnet, dass** an der Stirnfläche (20) ein Führungskanal (8) für eine Beobachtungsoptik und ein Führungskanal (10) für ein Beleuchtungsmittel münden.

4. Uretero-Renoskop nach einem der vorangehenden Ansprüche, **dadurch gekennzeichnet, dass** sich der Instrumentenkopf (6) zumindest an einem zwischen den keilförmigen Umfangsflächen (22, 24) angeordneten Umfangsabschnitt (26, 28) in distaler Richtung verjüngt.

5. Uretero-Renoskop nach einem der vorangehenden Ansprüche, **dadurch gekennzeichnet, dass** in einem Instrumentenkanal (12, 14) eine Lasersonde geführt ist, welche an einem sich proximalseitig an den Schaft (4) angrenzenden Steuerungsgehäuse (2) festlegbar ist.

6. Uretero-Renoskop nach Anspruch 5, **dadurch gekennzeichnet, dass** die Lasersonde in dem Instrumentenkanal (12, 14) in Längsrichtung des Instrumentenkanals (12, 14) begrenzt verschiebbar ist.

7. Uretero-Renoskop nach einem der vorangehenden Ansprüche, **dadurch gekennzeichnet, dass** mindestens ein Instrumentenkanal (12, 14) einen Spülkanal bildet.

8. Uretero-Renoskop nach einem der vorangehenden Ansprüche, **dadurch gekennzeichnet, dass** ein Instrumentenkanal (12, 14) einen Spülflüssigkeitszulauf und ein zweiter Instrumentenkanal (12, 14) einen Spülflüssigkeitsablauf bildet.

## Claims

1. A uretero-renoscope with a shaft (4), on whose distal end an instrument head (6) is arranged, which in the distal direction continuously tapers in a wedge-like manner at two peripheral sections (22, 24) essentially diametrically facing away from each other, **characterized in that** an instrument channel (12, 14) runs out at both peripheral sections (22, 24) formed beveled in a wedge-like manner.

2. A uretero-renoscope according to claim 1, **characterised in that** an end-face (20) is formed on the distal end of the instrument head (6).

3. A uretero-renoscope according to one of the preceding claims, **characterised in that** a guide channel (8) for observation optics and a guide channel (10) for illumination means run out at the end-face (20).

4. A uretero-renoscope according to one of the preceding claims, **characterised in that** the instrument head (6) tapers in the distal direction, at least on a peripheral section (26, 28) arranged between the wedge-like peripheral sections (22, 24).

5. A uretero-renoscope according to one of the preceding claims, **characterised in that** a laser probe is guided in one instrument channel (12, 14) which may be fixed on a control housing (2) bordering the shaft (4) on the proximal side.

6. A uretero-renoscope according to claim 5, **characterised in that** the laser probe is displaceable in the instrument channel (12, 14) in a limited manner in the longitudinal direction of the instrument channel (12, 14).

7. A uretero-renoscope according to one of the preceding claims, **characterised in that** at least one instrument channel (12, 14) forms a rinsing channel.

8. A uretero-renoscope according to one of the preceding claims, **characterised in that** one instrument channel (12, 14) forms a rinsing fluid feed and a second instrument channel (12, 14) forms a rinsing fluid discharge.

## Revendications

1. Urétérorénoscope comportant une tige (4) au niveau de l'extrémité distale de laquelle est disposée une tête d'instrument (6) qui se rétrécit en forme de coin de façon continue dans la direction distale au niveau de deux segments périphériques (22, 24) essentiellement diamétralement écartés l'un de l'autre, **caractérisé en ce qu'**au niveau des deux surfaces périphériques (22, 24) exécutées de façon biseautée en forme de coin débouche respectivement un canal d'instrument (12, 14).

2. Urétérorénoscope selon la revendication 1, **caractérisé en ce qu'**au niveau de l'extrémité distale de la tête d'instrument (6) est formée une surface frontale (20).

3. Urétérorénoscope selon l'une des revendications précédentes, **caractérisé en ce qu'**au niveau de l'extrémité frontale (20) débouche un canal de guidage (8) d'un instrument optique d'observation et un canal de guidage (10) d'un moyen d'éclairage.

4. Urétérorénoscope selon l'une des revendications précédentes, **caractérisé en ce que** la tête d'instrument (6) se rétrécit dans la direction distale au moins au niveau d'un segment périphérique (26, 28) disposé entre les surfaces périphériques (22, 24) en forme de coin.

5. Urétérorénoscope selon l'une des revendications précédentes, **caractérisé en ce que** dans un canal d'instrument (12, 14) est guidée une sonde laser qui peut être fixée au niveau d'un boîtier de commande (2) contigu à la tige (4) côté proximal.

6. Urétérorénoscope selon la revendication 5, **caractérisé en ce que** la sonde laser peut être déplacée de façon limitée dans le canal d'instrument (12, 14), dans la direction longitudinale du canal d'instrument (12, 14).

7. Urétérorénoscope selon l'une des revendications précédentes, **caractérisé en ce qu'**au moins un canal d'instrument (12, 14) forme un canal de rinçage.

8. Urétérorénoscope selon l'une des revendications précédentes, **caractérisé en ce qu'**un canal d'instrument (12, 14) forme une entrée de fluide de rinçage et **en ce qu'**un second canal d'instrument (12, 14) forme une sortie de fluide de rinçage.
